Europäisches Patentamt

European Patent Office

Office européen des brevets

(11) Numéro de publication: **0 455 547 B1**

(12) FASCICULE DE BREVET EUROPEEN

(45) Date de publication de fascicule du brevet: **19.10.94** (51) Int. Cl.5: **C07C 17/00**, C07C 1/26

(21) Numéro de dépôt: **91401121.8**

(22) Date de dépôt: **26.04.91**

(54) **Procédé de déchloration des chlorométhanes supérieurs.**

(30) Priorité: **03.05.90 FR 9005567**

(43) Date de publication de la demande:
**06.11.91 Bulletin 91/45**

(45) Mention de la délivrance du brevet:
**19.10.94 Bulletin 94/42**

(84) Etats contractants désignés:
**AT BE CH DE DK ES FR GB GR IT LI LU NL SE**

(56) Documents cités:
**US-A- 3 579 596**

**Ullman's Encyclopedia of Industrial Chemistry, 5. édition, vol. A13, p.228, 229**

**Kirk-Othmer, Encyclopedia of Chemical Technology, 3. edition, vol. 18, p. 308**

(73) Titulaire: **ELF ATOCHEM S.A.**
**4 & 8, Cours Michelet**
**La Défense 10**
**F-92800 Puteaux (FR)**

(72) Inventeur: **Correia, Yves**
**Les Lauzières**
**F-04160 Chateau-Arnoux (FR)**
Inventeur: **Nowocien, Joseph**
**23 Rue de la Garrigue**
**F-04160 Chateau-Arnoux (FR)**

EP 0 455 547 B1

**Description**

La présente invention est un procédé de déchloration de chlorométhanes supérieurs, c'est-à-dire du chloroforme ($CHCl_3$) et du tétrachlorure de carbone ($CCl_4$).

Les procédés de synthèse des chlorométhanes à l'échelle industrielle sont basés sur la chloration du méthane ou du chlorure de méthyle ($CH_3Cl$) et comme cette réaction est facile, ils ne sont pas complètement sélectifs. Par exemple, on ne sait pas produire du chlorure de méthylène ($CH_2Cl_2$) sans produire du chloroforme et du tétrachlorure de carbone.

Les chlorométhanes sont des intermédiaires de synthèse et si par exemple on utilise seulement le chloroforme, on ne sait pas quoi faire du tétrachlorure de carbone. Il est donc utile de disposer de procédés pour déchlorer un chlorométhane pour faire un produit moins chloré ou même du méthane.

Le brevet US 3 579 596 décrit la réaction du tétrachlorure de carbone ou du chloroforme en phase gazeuse avec de l'hydrogène sur un catalyseur au platine. Selon cet art antérieur, le tétrachlorure de carbone est rétrogradé en chloroforme et en méthane. En opérant selon ce procédé, on constate un encrassement du catalyseur et une instabilité de la température de réaction.

Une réaction semblable est décrite par ALVIN H. WEISS, BALJIT SINGH GAMBHIR et RICHARD B. LEON dans "Hydrodechlorination of carbon tetrachloride", journal of catalysis 22 (1971) page 245-254.

On a maintenant trouvé qu'on pouvait effectuer la déchloration des chlorométhanes supérieurs à l'aide d'hydrogène et d'oxygène sur un catalyseur et que la réaction était stable en température et sans encrassement du catalyseur.

La présente invention est donc un procédé de déchloration de chlorométhanes supérieurs à l'aide d'hydrogène et d'un catalyseur à base d'un métal choisi parmi le cuivre, l'or, l'argent ou le groupe du platine caractérisé en ce qu'on opère en présence d'oxygène.

Le procédé de l'invention permet de déchlorer du tétrachlorure de carbone pour produire du chloroforme et du méthane ou du chloroforme pour produire du chlorure de méthylène et du méthane. On ne sortirait pas du cadre de l'invention en effectuant la déchloration d'un mélange de chlorométhanes supérieurs. En opérant sur le mélange, on réussit à déchlorer la plus grande partie du tétrachlorure de carbone. Avantageusement, on effectue le procédé de l'invention sur un chlorométhane supérieur en phase gazeuse.

On peut utiliser de l'oxygène ou un gaz contenant de l'oxygène pourvu que les composants de ce gaz, autres que l'oxygène, soient inertes dans les conditions de réaction de la présente invention. On utilise avantageusement l'air.

Le catalyseur peut être constitué par un métal déposé sur un support. Le métal est choisi parmi les métaux de la colonne du cuivre, c'est-à-dire le cuivre, l'argent et l'or et parmi les métaux précieux de la colonne 8, c'est-à-dire le ruthénium, le rhodium, le palladium, l'osmium, l'iridium et le platine ; il peut être constitué d'un mélange de ces métaux.

Avantageusement, le support est constitué par des oxydes d'aluminium et de titane de surface comprise entre 20 et 300 $m^2$ par gramme. La teneur en métal est habituellement comprise entre 0,05 et 5 %, de préférence 0,1 à 1 % en poids de l'ensemble du catalyseur, c'est-à-dire du métal et du support. On peut mettre en oeuvre le procédé de l'invention en faisant passer un courant gazeux de chlorométhane, d'hydrogène et d'oxygène (ou d'air) sur un catalyseur en lit fixe ou en lit fluide. Il suffit ensuite d'un simple refroidissement à l'aide d'un échangeur pour séparer (i) l'hydrogène et l'oxygène (ou l'air) qui n'ont pas réagi ainsi que le méthane et l'acide chlorhydrique (HCl) formés au cours de la réaction et (ii) les hydrocarbures chlorés.

Par exemple si on applique le procédé de l'invention à du tétrachlorure de carbone, les hydrocarbures chlorés peuvent être du tétrachlorure de carbone non réagi, du chloroforme du chlorure de méthylène du perchloréthylène ($C_2Cl_4$) de l'hexachloroéthane ($C_6Cl_6$) et du chlorure de méthyle ($CH_3Cl$). On peut ensuite séparer en partie ou en totalité ces hydrocarbures chlorés, puis recycler le tétrachlorure de carbone en mélange avec de l'hydrogène et de l'oxygène dans le procédé de l'invention.

La réaction peut être conduite sous pression réduite ou sous des pressions égales ou supérieures à la pression atmosphérique, jusqu'à 10 bars. La température de la réaction est avantageusement comprise entre 30 et 350 °C et de préférence entre 100 et 250 °C.

Le rapport molaire $H_2$/chlorométhanes supérieurs est supérieur à l'unité et de préférence compris entre 1 et 10. L'oxygène qui peut être envoyé sous forme d'air, est avantageusement compris entre 0,01 et 5 % en moles par rapport à l'hydrogène. Le temps de séjour défini comme le rapport du volume de catalyseur au débit des réactifs hydrogène plus chlorométhanes supérieurs plus oxygène ou gaz contenant l'oxygène exprimé dans les conditions normales (pression atmosphérique et 0 °C), est compris entre 0,1 et 10 secondes et de préférence entre 0,5 et 5.

2

EP 0 455 547 B1

## EXEMPLE

Un réacteur est constitué d'un tube vertical de verre à double enveloppe, le tube intérieur a un diamètre intérieur de 26 mm et une hauteur de 850 mm. On dispose dans le tube intérieur, en partant du haut, 600 mm d'anneaux en verre 5 mm X 5 mm, puis 110 mm de catalyseur et enfin, 60 mm des anneaux de verre précédents.

Le réacteur est alimenté en réactifs par le haut. A l'intérieur du catalyseur on a disposé 3 sondes de température T1 à 20 mm en dessous du niveau haut de catalyseur, T2 30 mm sous T1, c'est-à-dire à 50 mm en dessous du niveau haut de catalyseur et T3 50 mm sous T2, c'est-à-dire à 100 mm en dessous du niveau haut de catalyseur. En sortie du réacteur on a disposé un condenseur à -30°C pour retenir les hydrocarbures chlorés, ensuite les gaz sont lavés à l'eau pour piéger HCl enfin les gaz restants passent dans un compteur.

Le réacteur ci-dessus est chargé avec un catalyseur à 0,5 % de platine déposé sur une alumine de 125 m²/g, on introduit un mélange d'hydrogène de CCl₄ et d'air.

Les conditions opératoires et les résultats sont rassemblés dans le tableau 1.

A titre d'exemple comparatif, on procède de la manière suivante :

- On interrompt l'introduction d'air et on constate une décroissance du taux de conversion du tétrachlorure de carbone, de la productivité du lit et la montée du perchloréthylène (PER) et de l'hexachloroéthane (Hexa). Les conditions opératoires et les résultats sont groupés au bas du tableau 1.
- Sur un catalyseur neuf, on procède à un essai dans les conditions identiques mais en absence d'air. Les conditions opératoires et les résultats sont rassemblés dans le tableau 2.

On constate, à travers ces essais, que l'ajout d'oxygène permet d'éviter l'encrassement du catalyseur et un meilleur contrôle de la température ; l'oxygène est presque entièrement retrouvé sous forme d'eau.

Dans les tableaux 1 et 2, le temps de séjour est exprimé comme le rapport de volume du catalyseur au volume des réactifs, N₂, CCl₄, O₂, N₂ dans les conditions normales TPN.

Dans les tableaux, la température du bain est la température du fluide circulant dans la double enveloppe, le rapport H₂/CCl₄ est un rapport molaire et l'air est exprimé en % en volume de l'hydrogène introduit.

3

TABLEAU 1

| H. de marche cumulées | Bilans températures | | | | Conditions | | | Taux conversion $CCl_4$ | Produc. g/ $CCl_4$ réagi/h lit/cata | Sélectivité/CCl4 réagi | | | | | |
|---|---|---|---|---|---|---|---|---|---|---|---|---|---|---|---|
| | Bain °C | T1 | T2 | T3 | Molaire $H_2$/$CCl_4$ | Air/$H_2$ % vol. | Temps séjour seconde | | | $HCCl_3$ % | $CH_4$ % | $CH_2Cl_2$ % | PER % | Hexa % | $CH_3Cl$ % |
| 9 | 38 | 169 | 73 | 42 | 8,3 | 2,5 | 1,75 | 80,1 | 1190 | 59,6 | 37,8 | 1,3 | 0,4 | 0,06 | 0,53 |
| 13 | 60 | 145 | 85 | 62 | 8,4 | 0,56 | 1,79 | 72,3 | 1062 | 67,7 | 30,1 | 1,05 | 0,5 | 0,3 | 0,37 |
| 83 | 60 | 160 | 95 | 65 | 8,65 | 5 | 1,72 | 33,6 | 1048 | 58,2 | 38,6 | 1,07 | 0,09 | 0,03 | 1,96 |
| 111 | 60 | 160 | 97 | 67 | 8,2 | 5 | 1,71 | 72,2 | 1085 | 57,7 | 38,9 | 1,06 | 0,08 | 0,07 | 2,27 |
| 156 | 60 | 159 | 95 | 66 | 8,5 | 5 | 1,72 | 71,5 | 1035 | 57,5 | 39,4 | 0,95 | 0,08 | 0,05 | 2,01 |
| 204 | 60 | 157 | 93 | 66 | 8,4 | 5 | 1,72 | 70,1 | 1030 | 58,1 | 38,7 | 0,77 | 0,03 | 0,06 | 2,39 |
| 259 | 60 | 158 | 92 | 66 | 8,5 | 5 | 1,72 | 70,2 | 1019 | 57,6 | 38,3 | 0,8 | 0,03 | 0,05 | 2,5 |
| 300 | 60 | 158 | 91 | 66 | 8,6 | 5 | 1,72 | 70,3 | 1010 | 57,1 | 39,1 | 0,9 | 0,07 | 0,05 | 2,9 |
| 325 | 120 | 153 | 151 | 130 | 8,3 | 0 | 1,79 | 83,4 | 1235 | 72,0 | 25,0 | 0,9 | 1,93 | 0,19 | - |
| 372 | 118 | 130 | 133 | 138 | 8 | 0 | 1,79 | 48,2 | 743 | 77,2 | 18,5 | 0,68 | 1,77 | 1,84 | - |
| 392 | 140 | 140 | 152 | 155 | 8 | 0 | 1,79 | 42,7 | 657 | 68,3 | 22,6 | 0,93 | 3,69 | 4,45 | - |

TABLEAU 2

| H. de marche cumulées | Bilans températures | | | | Conditions | | | | | Sélectivité/CCl4 réagi | | | | |
|---|---|---|---|---|---|---|---|---|---|---|---|---|---|---|
| | Bain °C | T1 | T2 | T3 | $\frac{H_2}{CCl_4}$ molaire | $\frac{Air}{H_2}$ % vol. | Temps séjour seconde | Taux conversion CCl4 | Produc. g/CCl4 réagi/h/lit/cata | HCCl3 % | CH4 % | CH2Cl2 % | PER % | Hexa % |
| 3,30 | 100 | 188 | 180 | 112 | 8,0 | 0 | 1,85 | 90,8 | 1471 | 68,7 | 28 | 1,95 | 1,3 | 0,05 |
| 21 | 100 | 154 | 162 | 123 | 8,0 | 0 | 1,85 | 90,5 | 1409 | 74,7 | 23,4 | 1,4 | 0,5 | 0,03 |
| 48 | 90 | 115 | 102 | 94 | 8,0 | 0 | 1,85 | 34,1 | 517,2 | 80,8 | 16,1 | 0,2 | 1,0 | 1,9 |
| 69 | 120 | 157 | 158 | 160 | 8,0 | 0 | 1,85 | 87,7 | 1317 | 73,5 | 23,8 | 1,3 | 1,3 | 0,14 |
| 93 | 120 | 148 | 147 | 153 | 8,0 | 0 | 1,85 | 78,1 | 1203 | 75,8 | 21,3 | 1,0 | 1,4 | 0,33 |
| 117 | 120 | 144 | 140 | 145 | 8,0 | 0 | 1,85 | 63,9 | 1000 | 77,8 | 19,2 | 0,73 | 1,5 | 0,76 |
| 141 | 130 | 153 | 149 | 152 | 8,0 | 0 | 1,85 | 58,5 | 939 | 73,1 | 20,5 | 0,86 | 3,08 | 2,45 |
| 166 | 130 | 148 | 143 | 142 | 8,0 | 0 | 1,85 | 40,8 | 653 | 73 | 21,5 | 0,30 | 2,47 | 2,8 |
| 189 | 160 | 176 | 173 | 170 | 8,0 | 0 | 1,85 | 35,8 | 594 | 55 | 25,5 | 1,36 | 5,9 | 12,22 |

**Revendications**

1. Procédé de déchloration de chlorométhanes supérieurs à l'aide d'hydrogène et d'un catalyseur à base d'un métal choisi parmi le cuivre, l'or, l'argent ou le groupe du platine caractérisé en ce qu'on opère en présence d'oxygène.

**2.** Procédé selon la revendication 1, caractérisé en ce que le chlorométhane supérieur est le tétrachlorure de carbone.

**3.** Procédé selon la revendication 1 ou 2, caractérisé en ce que le catalyseur est constitué par ledit métal déposé sur un support en oxyde d'aluminium ou de titane de surface comprise entre 20 et 300 $m^2$/g.

**4.** Procédé selon la revendication 3, caractérisé en ce que la teneur en métal est comprise entre 0,05 et 5 % en poids de l'ensemble métal plus support, et de préférence 0,1 à 1 %.

**5.** Procédé selon l'une des revendications 1 à 4, caractérisé en ce que la température est comprise entre 30 et 350 °C, et de préférence 100 et 250 °C.

**6.** Procédé selon l'une des revendications 1 à 5, caractérisé en ce que le temps de séjour est compris entre 0,1 et 10 secondes.

**7.** Procédé selon l'une des revendications 1 à 6, caractérisé en ce que le rapport molaire $H_2$ sur chlorométhane supérieur est supérieur à 1, et de préférence compris entre 1 et 10.

**8.** Procédé selon l'une des revendications 1 à 7, caractérisé en ce que le rapport oxygène sur hydrogène en mole est compris entre 0,01 et 5 %.

**9.** Procédé selon l'une des revendications 1 à 8, caractérisé en ce que le métal est le platine.

## Claims

**1.** Process for the dechlorination of higher chloromethanes using hydrogen and a catalyst based on a metal chosen from copper, gold, silver or the platinium group, characterized in that the dechlorination is carried out in the presence of oxygen.

**2.** Process according to Claim 1, characterized in that the higher chloromethane is carbon tetrachloride.

**3.** Process according to Claim 1 or 2, characterized in that the catalyst consists of the said metal deposited on a titanium oxide or aluminium oxide support with a surface of between 20 and 300 $m^2$/g.

**4.** Process according to Claim 3, characterized in that the metal content is between 0.05 and 5 % by weight of the combined metal plus support, and preferably 0.1 to 1 %.

**5.** Process according to one of Claims 1 to 4, characterized in that the temperature is between 30 and 350 °C, and preferably 100 and 250 °C.

**6.** Process according to one of Claims 1 to 5, characterized in that the residence time is between 0.1 and 10 seconds.

**7.** Process according to one of Claims 1 to 6, characterized in that the $H_2$ to higher chloromethane molar ratio is greater than 1, and preferably between 1 and 10.

**8.** Process according to one of Claims 1 to 7, characterized in that the oxygen to hydrogen ratio, in moles, is between 0.01 and 5 %.

**9.** Process according to one of Claims 1 to 8, characterized in that the metal is platinum.

## Patentansprüche

**1.** Verfahren zur Dechlorierung von höheren Chlormethanen mit Hilfe von Wasserstoff und einem Katalysator auf Metallbasis, der aus Kupfer, Gold, Silber oder der Platingruppe ausgewählt wird, dadurch gekennzeichnet, daß in Gegenwart von Sauerstoff gearbeitet wird.

2. Verfahren nach Anspruch 1, dadurch gekennzeichnet, daß das höhere Chlormethan Tetrachlorkohlenstoff ist.

3. Verfahren nach Anspruch 1 oder 2, dadurch gekennzeichnet, daß der Katalysator aus dem Metall besteht, das auf einem Träger aus Aluminiumoxid oder Titan mit einer Oberfläche im Bereich von 20 bis 300 $m^2$/g abgeschieden wird.

4. Verfahren nach Anspruch 3, dadurch gekennzeichnet, daß der Metallgehalt im Bereich von 0,05 bis 5 Gew.% und vorzugsweise von 0,1 bis 1 Gew.%, bezogen auf die Gesamtmenge an Metall plus Träger, liegt.

5. Verfahren nach einem der Ansprüche 1 bis 4, dadurch gekennzeichnet, daß die Temperatur im Bereich von 30 °C bis 350 °C und vorzugsweise im Bereich von 100 °C bis 250 °C liegt.

6. Verfahren nach einem der Ansprüche 1 bis 5, dadurch gekennzeichnet, daß die Aufenthaltsdauer im Bereich von 0,1 bis 10 Sekunden liegt.

7. Verfahren nach einem der Ansprüche 1 bis 6, dadurch gekennzeichnet, daß das Molverhältnis von $H_2$ zum höheren Chlormethan über 1 und vorzugsweise im Bereich von 1 bis 10 liegt.

8. Verfahren nach einem der Ansprüche 1 bis 7, dadurch gekennzeichnet, daß das Molverhältnis von Sauerstoff zu Wasserstoff im Bereich von 0,01 bis 5 % liegt.

9. Verfahren nach einem der Ansprüche 1 bis 8, dadurch gekennzeichnet, daß das Metall Platin ist.